# EUROPÄISCHE PATENTSCHRIFT

(11) **EP 0 641 179 B1**
(45) Veröffentlichungstag und Bekanntmachung des Hinweises auf die Patenterteilung: **22.07.1998**
(21) Anmeldenummer: 93905132.2
(22) Anmeldetag: 15.03.1993
(51) Int. Cl.: A61B 17/70

(54) **HAKEN MIT SCHRAUBE FÜR DIE BEHANDLUNG VON WIRBELSÄULENDEFORMITÄTEN**
HOOK WITH SCREW FOR TREATING SPINAL DEFORMITIES
CROCHET A VIS POUR TRAITER DES DEFORMATIONS DE LA COLONNE VERTEBRALE

(43) Veröffentlichungstag der Anmeldung: 08.03.1995
(73) Patentinhaber: SYNTHES AG, Chur, 7002 Chur (CH)
(72) Erfinder: SCHLÄPFER, Johannes, Fridolin, CH-8750 Glarus (CH)
(74) Vertreter: Lusuardi, Werther Giovanni, Dr.
(86) Internationale Anmeldenummer: CH9300070
(87) Internationale Veröffentlichungsnummer: WO9421186

(56) Entgegenhaltungen:
- EP-A- 0 348 272
- EP-A- 0 517 059
- FR-A- 2 151 475
- FR-A- 2 642 642

## Beschreibung

Die Erfindung bezieht sich auf einen Haken, gemäss dem Oberbegriff des Patentanspruchs 1.

Oft ist es notwendig, Deformitäten der Brustwirbelsäule operativ zu behandeln. Dabei muss die Anatomie der Wirbelsäule wieder hergestellt und aufrecht erhalten werden. Dazu werden Fixationssysteme entweder von dorsal oder ventral eingesetzt. Dorsale Systeme bestehen generell aus mindestens einem Längsträger, meistens aber ein linker und ein rechter, und einzelnen Verankerungselementen. Die letzteren bilden eine kraftübertragende Verbindung zwischen der Wirbelsäule und den Längsträgern. Derzeit sind drei verschiedene Möglichkeiten bekannt um die Längsträger mit der Wirbelsäule zu verbinden: Drahtcerclagen, Pedikelschrauben und Wirbelsäulenhaken.

Die Drahtcerclagen werden um die Lamina und durch die Dornfortsätze der Wirbelkörper gelegt. Das Anlegen von Drahtcerclagen ist allerdings mit einem gewissen Risiko für den Patienten verbunden, weil es zu Verletzungen des Rückenmarks kommen kann. Dazu kommt, dass die Drähte mit der Zeit durch den Knochen schneiden (hohe lokale Spannungsspitzen bewirken eine Knochenresorption unter dem Draht) oder einfach brechen.

Die Pedikelschrauben werden generell durch die Pedikel in die Wirbelkörper eingedreht. Die Pedikel sind in der oberen Brustwirbelsäule sehr schmal und aufgrund der Deformität schwierig zu lokalisieren. Dadurch ist der Einsatz von Pedikelschrauben in diesem Bereich risikoreich. Dazu kommt, dass sich viele Chirurgen generell scheuen, Pedikelschrauben im Bereich der Brustwirbelsäule einzusetzen.

Die Wirbelsäulenhaken sind die derzeit am häufigsten eingesetzten Elemente. Sie werden je nach Situation an der Lamina, beim Pedikel oder am Querfortsatz eingehängt. Sie bieten gegenüber den Drahtcerclagen den Vorteil, dass die Belastung über eine relativ grosse Fläche vom Knochen auf den Haken übertragen wird und dementsprechend kaum eine Knochenresorption an der Berührungsstelle zwischen Knochen und Haken stattfindet. Die Haken haben aber gegenüber den Pedikelschrauben den grossen Nachteil, dass sie nur Belastungen auf die Längsträger übertragen können, wenn sie fest gegen den Knochen gedrückt werden. Bei den derzeitigen Systemen kann eine solche Vorlast nur über die Längsträger aufgebracht werden. Damit wird es sehr schwierig, die Wirbel im Deformationsbereich mit den heute bekannten Haken einzeln zu manipulieren.

Aus der FR-A-2 642 642 ist ein Haken gemäss dem Oberbegriff des Anspruchs 1 bekannt, welcher eine Bohrung zur Aufnahme einer Schraube aufweist. Die Bohrung ist bei diesem bekannten Implantat in einem aus dem Schaft des Hakens hervortretenden, parallel zum Längsträger verlaufenden Vorsprung angebracht.

Hier will die Erfindung Abhilfe schaffen. Der Erfindung liegt die Aufgabe zugrunde, einen Haken zu schaffen, der unabhängig von dem ihn tragenden Längsträger in stabiler Weise fest mit dem Wirbel verbunden ist.

Die Erfindung löst die gestellte Aufgabe mit einem Haken, welcher die Merkmale des Anspruchs 1 aufweist.

Der erfindungsgemässe Pedikelhaken eignet sich insbesondere für die thorakale Anwendung. Dank der zusätzlichen, durch die Hakenklinge einführbare Schraube kann der Pedikelhaken fest in den knöchernen Sitz gepresst werden. Die Schraube führt dabei von kaudal nach kranial (unter einem Winkel von etwa 120° - 125° zur Längsachse des Pedikelhakens) durch die Hakenklinge in die Hauptmasse des zum entsprechenden Wirbel gehörenden Fazettengelenkes hinein. Dadurch kann der Pedikelhaken ähnlich zu einer Pedikelschraube Kräfte und Momente aufnehmen ohne aus seiner knöchernen Verankerung herauszurutschen, was die Voraussetzung für die Durchführung segmentaler Korrekturen bildet.

Die weiteren durch die Erfindung erreichten Vorteile sind vielfältig:
- dank der unabhängigen, stabilen Verbindung des erfindungsgemässen Hakens mit dem Wirbel wird der Haken einzeln manipulierbar;
- die zu behandelnde Wirbelsäulendeformität kann Schritt für Schritt von der weniger deformierten Seite her kommend korrigiert werden;
- die Montage des Hakens ist im thorakalen Bereich sicherer als das Einsetzen von Pedikelschrauben, da der Haken gleichzeitig als Bohrlehre für die Knochenschraube dient;
- der Haken kann aufgrund der schrägen Lage der Knochenschraube optimal in den knöchernen Sitz gedrückt werden.

Die Befestigung des erfindungsgemässen Hakens an einen Längsträger, innerhalb einer Wirbelsäulenfixationsvorrichtung, kann auf verschiedene, bekannte Arten erfolgen. Zu diesem Zweck weist der Schaftteil des erfindungsgemässen Hakens entsprechende Konstruktionselemente auf, welche die Verbindung zum Längsträger gestattet, beispielsweise wie in der EP-A 0 348 272 offenbart.

Die klinische Applikation des erfindungsgemässen Hakens ist analog zu derjenigen bekannter Systeme und ist im Detail in J.Dubousset und Y.Cotrel, Orthopäde (1989) 18:118-127 "Die CD-Instrumentation in der Behandlung von Wirbelsäulendeformitäten" beschrieben.

Ausführungsbeispiele der Erfindung, welche zugleich das Funktionsprinzip erläutern, sind in den Zeichnungen dargestellt und werden im folgenden näher beschrieben, wobei in allen Zeichnungen ein Pedikelhaken als Ausführungsbeispiel dargestellt ist.
Fig. 1 stellt einen Längsschnitt durch den erfindungsgemässen Pedikelhaken dar;
Fig. 2 stellt einen Teilschnitt durch den erfindungsgemässen Pedikelhaken mit eingesetzter Schraube dar;
Fig. 3 stellt einen Teilschnitt durch den erfindungsgemässen Pedikelhaken mit einer modifizierten Schraube dar; und
Fig. 4 stellt eine Seitenansicht auf den erfindungsgemässen, in die Wirbelknochen implantierten Pedikelhaken dar.

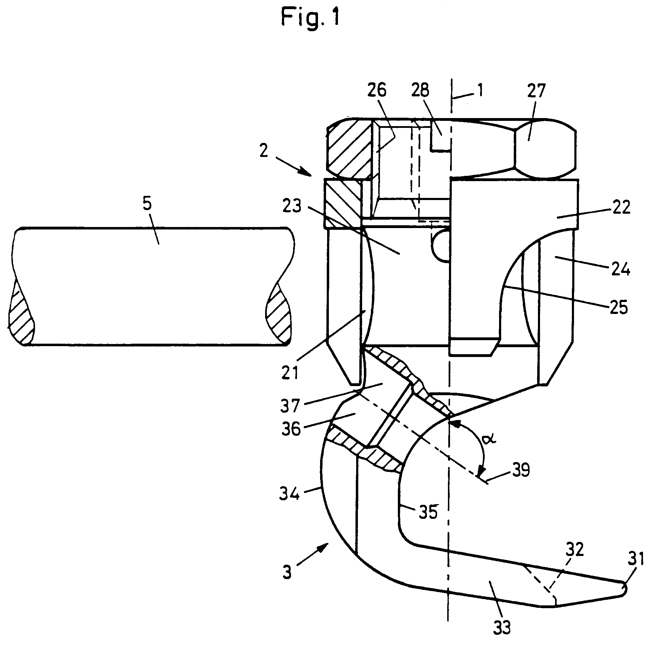

Der in Fig. 1 im Detail gezeigte Pedikelhaken besteht im wesentlichen aus einem an einen Längsträger 5 befestigbaren Schaftteil 2 und einer daran anschliessenden, gebogenen Hakenklinge 3.

Der Schaftteil 2 besteht aus einer Verbindungseinrichtung, welche in der unter der Nummer EP-A1 517 059 publizierten europ. Patentanmeldung näher beschrieben ist.
Sie besteht im wesentlichen aus einem äusseren, eine kreiszylindrische Bohrung 21 aufweisenden Element 22 und einem inneren, in der Bohrung 21 koaxial verschiebbaren und um seine Achse 1 rotierbaren zylindrischen Element 23. Beide Elemente 22 und 23, besitzen zwei gemeinsame quer zur Achsrichtung 1 der beiden Elemente 22 und 23 gebildete Durchgangsöffnungen 24 und 25 zur Aufnahme des Längsträgers 5. Wird der Längsträger 5 in die beiden Bohrungen 24 und 25 eingeführt, so wird die axiale Verschiebbarkeit des inneren Elementes 23 in der Bohrung 21 des äusseren Elementes 22 in einer Richtung blockiert und seine Rotation auf den Winkelbereich der Durchgangsöffnungen 24 und 25 der beiden Elemente 22 und 23 eingeschränkt. Das innere Element 23 ist wegen seiner Durchgangsöffnung 25 gegenüber seiner Achse 1 elastisch deformierbar und weist im weiteren ein Aussengewinde 26 auf, welches mit der Mutter 27 korrespondiert. Bei einer axialen Verspannung des inneren Elementes 23 mittels der Mutter 27, welche eine axiale Verschiebung des inneren Elementes 23 relativ zum äusseren Element 22 bewirkt, erfolgt deshalb eine Aufweitung des elastisch deformierbaren, inneren Elementes 23 und ein Verspannung desselben innerhalb der Bohrung 21 des äusseren Elementes 22 bei gleichzeitiger Blockierung der gesamten Verbindungseinrichtung und des darin eingeführten Längsträgers 5. Das Anziehen, bzw. Lösen der Mutter 27 geschieht mittels zweier zeichnerisch nicht dargestellter Instrumente, eines Steckschlüssels und eines geeigneten Instrumentes, das beim Manipulieren der Mutter 27 den Pedikelhaken in Position hält. Dieses Instrument wird wie ein Schraubenzieher in den Längsschlitz 28 der Mutter 27 eingeführt.

Die Durchgangsöffnung 25 ist mit Vorteil derart ausgebildet, dass der Längsträger 5 nicht unbedingt parallel zur Hakenklinge 3 verlaufen muss. Dadurch wird verhindert, dass bei der Montage des Längsträger 5 der erfindungsgemässe Haken mit der Schraube 4 unnötig belastet wird, was die Gefahr in sich birgt, dass die Schraube 4 aus ihrer Verankerung im Knochen gerissen wird. Bei steifen Skoliosen ist oft eine 100-%-ige Korrektur unmöglich. In diesen Fällen ist es von Vorteil, Haken mit einer Durchgangsöffnung senkrecht (statt parallel) zum Längsträger 5 zu haben. Um dies zu realisieren wird die Bohrung 21 des Schaftteils 2 statt gleichsinnig zur Hakenklinge 3 im rechten Winkel dazu angeordnet zur Aufnahme eines Längsträger-Zwischenstücks (oder Querträgers) mit vergrössertem Profil, das eine Durchgangsöffnung zur Aufnahme des eigentlichen Längsträgers 5 aufweist. Mittels dieses Längsträger-Zwischenstücks ist es möglich den Längsträger 5 nicht nur zentral sondern auch in einem gewissen Abstand von der Längsachse 1 mit dem Haken zu verbinden. Die Durchgangsöffnung im Längsträger-Zwischenstück kann geschlossen oder offen gestaltet sein und kann derart ausgebildet werden, dass das Längsträger-Zwischenstück um einen bestimmten Betrag bezüglich der Hakenklinge 3 rotiert werden kann. Der Vorteil dieser - zeichnerisch nicht dargestellten - Ausführung liegt darin begründet, dass damit unnötige Kräfte während der Montage des Längsträger-Zwischenstücks verhindert werden kann.

Statt der oben beschriebenen, bevorzugten Verbindungseinrichtung für den Schaftteil 2 kann auch jedes andere bekannte Verbindungssystem verwendet werden um den Schaftteil 2 an einen Längsträger 1 zu fixieren, beispielsweise gemäss der EP-A1 348 272.

Die Hakenklinge 3 ist an ihrer Hinterseite 34 konvex und an ihrem freien Ende 31 zweischenklig ausgebildet. Die beiden Schenkel 33 bilden eine der Pedikelgeometrie angepasste Einbuchtung 32. Die Länge der beiden Schenkel 33 kann verschieden lang gewählt werden, wie dies im Detail in der unter der Nummer EP-A1 0 517 059 publizierten europ. Patentanmeldung beschrieben ist. Das abgebogene, freie Ende 31 der Hakenklinge 3 verläuft im wesentlichen senkrecht zur Längsachse 1.

An der konvexen Hinterseite 34 der Hakenklinge 3 ist eine sich zur konkaven Vorderseite 35 erstreckende, durchgehenden Bohrung 36 zur Aufnahme einer Schraube 4 vorgesehen. Die Längsachse 39 der Bohrung 36 schliesst vorzugsweise einen Winkel α von 125° - 130° mit der Längsachse 1 ein.

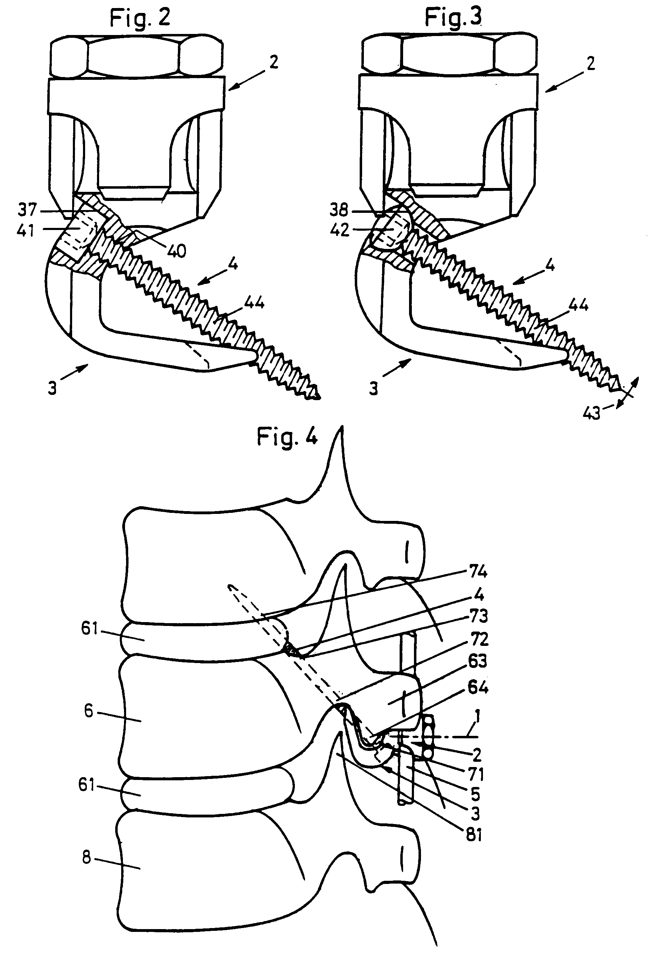

Wie in Fig. 2 dargestellt kann die Bohrung 36 einen zylindrischen Abschnitt 37 zur Aufnahme der mit einem zylindrischen Kopf 41 versehenen Schraube 4 aufweisen. Stattdessen kann - wie in Fig. 3 dargestellt - die Bohrung 36 auch einen sphärischen Abschnitt 38 zur Aufnahme einer mit einem sphärischen Kopf 42 versehenen Schraube 4 aufweisen. Die Ausführung mit einem sphärischen Abschnitt 38 gestattet innerhalb gewisser Grenzen eine durch den Pfeil 43 angedeutete räumliche Verschwenkbarkeit der Schraube 4, währenddem bei der Ausführung mit zylindrischem Abschnitt 37 die Schraube 4 geführt ist.

Die Bohrung 36 kann auch konisch ausgebildet sein mit einem ähnlichen Effekt wie bei der zylindrisch geführten Schraube 4 (Fig. 2).

Die Bohrung 36 kann wie in Fig. 2 dargestellt ein Gewinde 40 aufweisen, welches zumindest in der Steigung dem Gewinde 44 der Schraube 4 entspricht. In diesem Fall ist es möglich, die konkave Vorderseite 35 der Hakenklinge 3 in einem genau definierten Abstand vom Knochen zu halten. Ein weiterer Vorteil dieser Ausführung liegt darin, dass die Schraube 4 beim Anziehen im Haken verspannt wird. Dadurch wird verhindert, dass sich die Schraube 4 im Laufe der Zeit aus dem Knochen herausdreht. Bei anderen Ausführungsformen wird ein Herausdrehen der Schraube 4 dadurch verhindert, dass der Schraubenkopf (41;42) direkt unter dem Längsträger 5 plaziert wird.

Wie in Fig. 4 dargestellt wird der erfindungsgemässe Pedikelhaken zwischen das inferiore Fazettengelenk 64 des Wirbels 6 und das superiore Fazettengelenk 81 des durch die Bandscheibe 61 beabstandeten, darunterliegenden Wirbels 8 eingeschoben, wobei die Klinge 3 durch ihre gabelförmige Ausbildung den Pedikel 63 von kaudal her umfasst. Die Knochenschraube 4 ist im inferioren Fazettengelenk 64 des Wirbels 6 verankert und durchdringt, bzw. berührt je nach deren Länge bis zu vier kortikale Zonen (71,72,73,74).

Als Hauptindikation für den Gebrauch dieser Ausführungsform gilt die segmentale Korrektur bei der Skoliose. Das Ziel der Behandlung der Skoliose ist es, die Wirbelsäule zum Längsträger 5 hin zu bringen und die verdrehten Wirbel zurückzurotieren. Bei den Vorrichtungen gemäss dem Stand der Technik erfolgt dies derart, dass die Wirbelsäule in einem einzigen Schritt korrigiert wird, indem der Längsträger aus der Frontalebene in die Sagittalebene gedreht wird (sog. globale Korrektur). Bei dieser Korrekturmethode ist das auf die Wirbelsäule ausgeübte derotierende Moment sehr klein, da die kaudal, zum Apexwirbel liegenden Haken in die Lamina eingehängt werden müssen und somit praktisch über dem Drehzentrum liegen.

## Patentansprüche

1. Haken für die Behandlung von Wirbelsäulendeformitäten, mit
A) einem eine Längsachse (1) aufweisenden Schaftteil (2), an welchem quer zur Längsachse (1) ein Längsträger (5) befestigbar ist,
B) einer durchgehenden Bohrung (36) zur Aufnahme einer Schraube (4), und
C) einer am Schaftteil (2) anschliessenden, von der Längsachse (1) weggebogenen Hakenklinge (3) mit einer konvexen Hinterseite (34) und einer konkaven Vorderseite (35),
**dadurch gekennzeichnet, dass**
D) sich die Bohrung (36) von der konvexen Hinterseite (34) zur konkaven Vorderseite (35) der Hakenklinge (3) erstreckt.

2. Haken nach Anspruch 1, dadurch gekennzeichnet, dass die Längsachse (39) der Bohrung (36) einen Winkel α von 115° - 130°, vorzugsweise von 120° - 125° mit der Längsachse (1) des Schaftteils (2) einschliesst.

3. Haken nach Anspruch 1 oder 2, dadurch gekennzeichnet, dass die Bohrung (36) einen zylindrischen oder konischen Abschnitt (37) hat zur Aufnahme einer mit einem zylindrischen, beziehungsweise konischen Kopf (41) versehenen Schraube (4).

4. Haken nach Anspruch 1 oder 2, dadurch gekennzeichnet, dass die Bohrung (36) einen sphärischen Abschnitt (38) hat zur Aufnahme einer mit einem sphärischen Kopf (42) versehenen Schraube (4).

5. Haken nach einem der Ansprüche 1 - 4, dadurch gekennzeichnet, dass die Bohrung (36) mindestens teilweise ein Gewinde (39) aufweist, welches dem Gewinde (44) der Schraube (4) entspricht.

6. Haken nach einem der Ansprüche 1 - 5, dadurch gekennzeichnet, dass das abgebogene, freie Ende (31) der Hakenklinge (3) im wesentlichen senkrecht zur Längsachse (1) des Schaftteils (2) verläuft.

7. Haken nach einem der Ansprüche 1 - 6, dadurch gekennzeichnet, dass das freie Ende (31) der Hakenklinge (3) mit einer Einbuchtung (32) versehen ist, welche das freie Ende (3) der Hakenklinge (3) in zwei Schenkel (33) aufteilt.

8. Haken nach einem der Ansprüche 1 - 7, dadurch gekennzeichnet, dass der Schaftteil (2) eine Klemmvorrichtung (21,22,23,24,25, 26,27,28) aufweist mit welcher der Längsträger (5) senkrecht zur Längsachse (1) des Schaftteils (2) fixiert werden kann.

## Claims

1. Hook for the treatment of spinal deformities with
A) a shaft section (2) having a longitudinal axis (1) at which a longitudinal support (5) is attachable transverse to the longitudinal axis (1),
B) a through bore hole (36) for the acceptance of a screw (4), and
C) a hook blade (3) adjoining the shaft section (2) bent away from the longitudinal axis (1) with a convex back side (34) and a concave front side (35),
characterized in that
D) the bore hole (36) extends from the convex back side (34) to the concave front side (35) of the hook blade (3).

2. Hook according to claim 1, characterized in that the longitudinal axis (39) of the bore hole (36) includes an angle α with an amount of 115° - 130°, preferably with an amount of 120° - 125° with the longitudinal axis (1) of the shaft section (2).

3. Hook according to claim 1 or 2, characterized in that the bore hole (36) has a cylindrical or conical portion (37) for the acceptance of a screw (4) provided with a cylindrical respectively conical head (41).

4. Hook according to claim 1 or 2, characterized in that the bore hole (36) has a spherical portion (38) for the acceptance of a screw (4) provided with a spherical head (42).

5. Hook according to one of the claims 1 - 4, characterized in that the bore hole (36) provides at least partially a thread (39) corresponding to the thread (44) of the screw (4).

6. Hook according to one of the claims 1 - 5, characterized in that the bent off free end (31) of the hook blade (4) runs essentially perpendicular to the longitudinal axis (1) of the shaft section (2).

7. Hook according to one of the claims 1 - 6, characterized in that the free end (31) of the hook blade (3) is provided with a recess (32) which separates the free end (31) of the hook blade (3) into two legs (33).

8. Hook according to one of the claims 1 - 7, characterized in that the shaft section (2) provides a clamping device (21,22,23,24,25,26,27,28) by which means the longitudinal support (5) can be fastened perpendicular to the longitudinal axis (1) of the shaft section (2).

## Revendications

1. Crochet pour le traitement de déformations de la colonne vertébrale, comportant
A) un corps (2) présentant un axe longitudinal (1) et sur lequel un support longitudinal (5) peut être fixé transversalement par rapport à l'axe longitudinal (1),
B) un alésage (36) qui le traverse, pour la réception d'une vis (4), et
C) une lame de crochet (3) qui se raccorde au corps (2), s'incurve en s'éloignant de l'axe de longitudinal (1) et présente une face arrière (34) convexe et une face avant (35) concave,
caractérisé en ce que
D) l'alésage (36) s'étend entre la face arrière (34) convexe et la face avant (35) concave de la lame de crochet (3).

2. Crochet selon la revendication 1, caractérisé en ce que l'axe longitudinal (39) de l'alésage (36) forme avec l'axe longitudinal (1) du corps (2) un angle α de 115° à 130°, et de préférence de 120° à 125°.

3. Crochet selon les revendications 1 ou 2, caractérisé en ce que l'alésage (36) présente une partie cylindrique ou conique (37) pour la réception d'une vis (4) dotée d'une tête respectivement cylindrique ou conique (41).

4. Crochet selon les revendications 1 ou 2, caractérisé en ce que l'alésage (36) présente une partie sphérique (38) pour la réception d'une vis (4) dotée d'une tête sphérique (42).

5. Crochet selon l'une des revendications 1 à 4, caractérisé en ce que l'alésage (36) présente sur au moins une partie un filet (39) qui correspond au filet (44) de la vis (4).

6. Crochet selon l'une des revendications 1 à 5, caractérisé en ce que l'extrémité libre (31) incurvée de la lame (3) du crochet s'étend essentiellement à la perpendiculaire de l'axe longitudinal (1) du corps (2).

7. Crochet selon l'une des revendications 1 à 6, caractérisé en ce que l'extrémité libre (31) de la lame (3) du crochet est dotée d'une anse (32) qui partage l'extrémité libre (31) de la lame (3) du crochet en deux bras (33).

8. Crochet selon l'une des revendications 1 à 7, caractérisé en ce que le corps (2) présente un dispositif de serrage (21, 22, 23, 24, 25, 26, 27, 28) avec lequel le support longitudinal (5) peut être fixé perpendiculairement à l'axe longitudinal (1) du corps (2).
